# EUROPEAN PATENT SPECIFICATION

(11) **EP 2 432 551 B1**
(45) Date of publication and mention of the grant of the patent: **01.11.2017**
(21) Application number: 10720230.1
(22) Date of filing: 14.05.2010
(51) Int. Cl.: A61N 1/362, A61B 5/0452, A61B 5/0456, A61N 1/37, A61B 5/0215, A61B 5/024

(54) **IMPLANTABLE MEDICAL DEVICE FOR CARDIAC ELECTRICAL STIMULATION**
IMPLANTIERBARE MEDIZINISCHE VORRICHTUNG KARDIALE ELEKTROSTIMULATION
DISPOSITIF MÉDICAL IMPLANTABLE POUR STIMULATION ÉLECTRIQUE CARDIAQUE

(30) Priority: 19.05.2009 US 179452 P; 30.04.2010 US 771217
(43) Date of publication of application: 28.03.2012
(73) Proprietor: Medtronic, Inc., Minneapolis, MN 55432 (US)
(72) Inventor: SOMMER, John, L., Coon Rapids, MN 55448 (US); BRABEC, Scott, J., Elk River, MN 55330 (US); URBAN, Jon, F., Minneapolis, MN 55414 (US); YONG-FU, Xiao, Blaine, MN 55449 (US); ZHOU, Xiaohong, Woodbury, MN 55129 (US)
(74) Representative: Hughes, Andrea Michelle
(86) International application number: PCT/US2010/034825
(87) International publication number: WO 2010/135164

(56) References cited:
- US-A1- 2004 158 292
- US-A1- 2008 077 187
- US-B1- 7 024 243

## Description

### FIELD OF THE INVENTION

The present invention relates generally to implantable medical devices and more specifically to cardiac stimulation.

### BACKGROUND OF THE INVENTION

In the early days of implantable cardiac pacing, it was observed that paired pacing (two or more closely spaced pacing pulses delivered at the time-out of an escape interval) and triggered or coupled pacing (one or more pacing pulses delivered following the detection of a P-wave or R-wave terminating an escape interval) with relatively short inter-pulse intervals (150 to 250 milliseconds in dogs and about 300 milliseconds in human subjects) beneficially slowed heart rate and increased cardiac output. The result of the second pulse, applied within the relative refractory period of the first paced or spontaneous depolarization, is to prolong the refractory period and effect a slowing of the heart rate from its spontaneous rhythm without an attendant mechanical myocardial contraction. This slowing effect has been employed since that time in many applications, including the treatment of atrial and ventricular tachycardias, where a single pulse or a burst of pulses are coupled to a spontaneous tachycardia event with a coupling interval that is shorter than and can be set as a fraction of the tachycardia interval as taught, for example, in U.S. Patent Nos. 3,857,399 and 3,939,844. The slowing of the heart rate by coupled pacing is accompanied by the ability to increase or decrease the rate with subsequent coupled pacing within wide limits.

Paired and coupled stimulation of a heart chamber also cause a potentiation of contractile force effect through a phenomenon known as post-extrasystolic potentiation (PESP) described in detail in commonly assigned U.S. Patent No. 5,213,098. The force of contraction of the heart is increased during the heart cycle that the paired or coupled stimulation is applied, and the increase persists but gradually diminishes over a number of succeeding heart cycles. Other measurable PESP effects that also persist but gradually decline over a number of heart cycles include changes in the peak systolic blood pressure, the rate of contraction of the ventricular muscle with a resulting increase of the rate of rise of intra-ventricular pressure (dP/dt), an increase in coronary blood flow, and an increase in the oxygen uptake of the heart per beat. Investigators observed that PESP was accompanied by an increase in the myocardial oxygen consumption of 35% to 70% as compared with single pulse stimulation at the same rate and was associated with a significant improvement in ejection fraction. The addition of a third stimulus increased the myocardial oxygen uptake even further without any attendant observed increase in cardiac contractile force. The alterations in coronary flow roughly parallel the oxygen consumption of the heart as observed in such studies.

The marked potentiation effect produced by paired stimulation led certain investigators to speculate that PESP stimulation would be beneficial in treating heart failure in humans and conducted studies using the technique in the treatment of acute heart failure induced in dogs. Improvements in left ventricular performance and cardiac output produced by such paired pacing in these dogs were observed by several investigators. In other studies conducted on relatively normal dogs' hearts, it was confirmed that paired pacing offered no increase in cardiac output, most likely due to reflex compensation. Early investigators conducted a large number of animal and human studies employing paired and coupled stimulation of the atrial and ventricular chambers, and medical devices were made available by Medtronic, Inc. and other companies in an effort to employ the PESP effect. However, it was realized that the application of closely timed paired and coupled pacing pulses, particularly the high energy pacing pulses that were employed at that time in implantable pacemakers, could trigger a tachyarrhythmia in patient's hearts that were susceptible. The efforts to capitalize on the PESP effects were largely abandoned. A history of the investigations and studies conducted is set forth in the above-referenced 098 patent.

A series of PCT publications including, for example, PCT WO 97/25098 describe the application of one or more "non-excitatory" anodal or cathodal stimulation pulses to the heart and maintain that improvements in LV performance may be realized without capturing the heart. In a further commonly assigned U.S. Patent No. 5,800,464, sub-threshold anodal stimulation is provided to the heart to condition the heart to mechanically respond more vigorously to the conventional cathodal supra-threshold pacing pulses.

US 2008/0077187 teaches atrial pacing during the ventricular refractory period.

### SUMMARY OF THE INVENTION

Notwithstanding the above discussed work, there still remains a need for improved treatments for tachycardia, heart failure and hypertension. The present invention provides an apparatus to decrease heart rate and blood pressure as defined by claim 1. As discussed below, in a healthy heart, the sino-atrial node (SAN) initiates
action potentials which propagate to the whole heart via a conductive system. Compared with the ventricles, the SAN, atria and AV node (AVN) are normally excited earlier and recover earlier in the cardiac cycle than the ventricles. However, due to longer action potential durations associated with the ventricular myocardium, the refractory period of the ventricles continues after the end of the atrial and nodal refractory periods discussed above. Therefore, there is a stimulus time window in which the atria, SAN and/or AVN may be excited while the ventricles remain refractory to stimulation.

Excitation of the atria, SAN and/or AVN during the ventricular refractory period resets the atrial cycle (resets the SAN) and starts a new refractory period in the atrial and AV nodal tissue, without triggering a ventricular contraction. The result is that propagation of a depolarization to the ventricles effective to cause a contraction is delayed until the next subsequent depolarization of the SAN. The ventricular rate is thus reduced by up to 50% from the SA nodal rate. The inventors have determined that in conjunction with this reduction in ventricular rate, both the systolic and diastolic blood pressures may be reduced. The degree of reduction in rate and blood pressure varies with the timing of the atrial or nodal excitation stimulus relative to preceding atrial and ventricular depolarizations. The effect of the stimulation can thus be titrated against a desired reduction in rate or blood pressure.

The stimulus pulses may correspond to typical cardiac pacing pulses or may be pulse bursts having burst envelopes corresponding to the duration and/or morphology of normal cardiac pacing pulses. Longer pulse durations, e.g. 10 ms or more may also be employed. The particular form of the pulse is not critical so long as it triggers a depolarization of the desirred atrial or nodal tissue. Proper timing of the stimulus pulses within the defined excitation window is important to assure that the desired effect is produced without induction of arrhythmias.

The stimulus time window extends from the point at which the atrial or nodal tissue to be stimulated becomes non-refractory up to the point at which the stimulated atrial or nodal depolarization could propagate to the ventricles to cause a ventricular depolarization. The time window will vary from patient to patient, but generally will fall between the end of the atrial and/or nodal refractory periods and the end of the ventricular refractory period. The window generally thus extends within a time period occurring between about 80 ms to about 200 ms following a normally conducted ventricular depolarization. Refractory period timing and durations may vary with the individual and with the underlying heart rhythm, and thus the duration of the time window will also vary. Timing of the stimulus pulses may be based upon a preceding atrial depolarization, ventricular depolarization, or possibly both. Timing of the stimulus pulses may be predefined by the attending physician and programmed into the device or may be varied by the device in order to achieve a desired reduction in heart rate or blood pressure.

### BRIEF DESCRIPTION OF THE DRAWINGS

These and other advantages and features of the present invention will be more readily understood from the following detailed description of the preferred embodiments thereof, when considered in conjunction with the drawings, in which like reference numerals indicate identical structures throughout the several views, and wherein:
FIG. 1 is a drawing illustrating an implantable stimulator and associated lead system according to a first embodiment of the present invention.
FIG. 2 is a simplified block diagram of one embodiment of IPG circuitry and associated leads which may be employed in delivering heart stimulation therapy according to the various described embodiments of the present invention.
FIG. 3 is a functional flow chart illustrating the operation of a first embodiment of the present invention.
FIG. 4 is a functional flow chart illustrating the operation of a second embodiment of the present invention.
FIG. 5 is a functional flow chart illustrating details of operation of the first and second embodiments of the invention of Figures 3 and 4.
FIG. 6 is a drawing of a patient activator or programmer for triggering a stimulator as illustrated in Figures 1 and 2 to deliver a stimulation therapy according to the present invention.

### DETAILED DESCRIPTION OF THE PREFERRED EMBODIMENTS

In the following detailed description, references are made to illustrative embodiments for carrying out the invention. It is understood that other embodiments may be utilized without departing from the scope of the invention as defined by the claims.

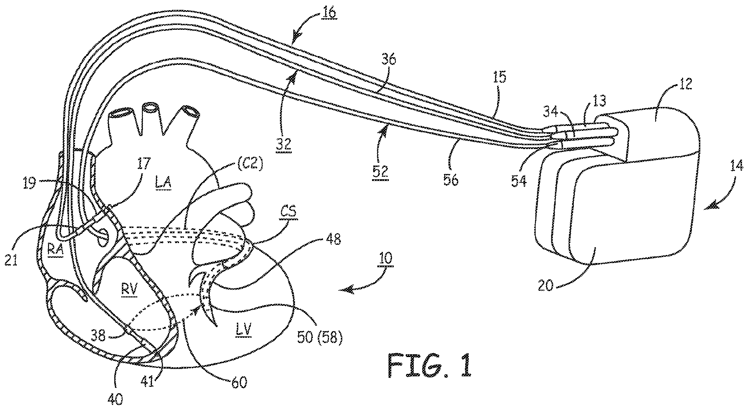

In FIG. 1, heart 10 includes the upper heart chambers, the right atrium (RA) and left atrium (LA), and the lower heart chambers, the right ventricle (RV) and left ventricle (LV) and the coronary sinus (CS) extending from the opening in the right atrium laterally around the atria to form the great vein that extends further inferiorally into branches of the great vein. The cardiac cycle commences normally with the generation of the depolarization impulse at the SA Node in the right atrial wall. The impulse then conducts through the right atrium by way of Internodal Tracts, and conducts to the left atrial septum by way of Bachmann's Bundle. The RA depolarization wave reaches the Atrio-ventricular (AV) node and the atrial septum within about 40 ms. and reaches the furthest walls of the RA and LA within about 70 ms. Approximately 50 ms. following electrical activation, the atria contract, the aggregate RA and LA depolarization wave appears as the P-wave of the PQRST complex when sensed across external ECG electrodes and displayed. The component of the atrial depolarization wave passing between a pair of unipolar or bipolar pace/sense electrodes, respectively, located on or adjacent the RA or LA is also referred to as a sensed P-wave. Although the location and spacing of the external ECG electrodes or implanted unipolar atrial pace/sense electrodes has some influence, the normal P-wave width does not exceed 80 ms. in width as measured by a high impedance sense amplifier coupled with such electrodes. A normal near field P-wave sensed between closely spaced bipolar pace/sense electrodes and located in or adjacent the RA or the LA has a width of no more than 60 ms. as measured by a high impedance sense amplifier.

The depolarization impulse that reaches the AV node conducts down the bundle of His in the intra-ventricular septum after a delay of about 120 ms. The depolarization wave reaches the apical region of the heart about 20 ms later and then travels superiorly through the Purkinje fiber network over the remaining 40 ms. The aggregate RV and LV depolarization wave and the subsequent T-wave accompanying re-polarization of the depolarized myocardium are referred to as the QRST portion of the PQRST cardiac cycle complex when sensed across external ECG electrodes and displayed. When the amplitude of the QRS ventricular depolarization wave passing between a bipolar or unipolar pace/sense electrode pair located on or adjacent to the RV or LV exceeds a defined threshold amplitude, it is detected as a sensed R-wave. Although the location and spacing of the external ECG electrodes or implanted unipolar ventricular pace/sense electrodes has some influence on R-wave sensing, the normal R-wave duration does not exceed 80 ms. as measured by a high impedance sense amplifier. A normal near field R-wave sensed between closely spaced bipolar pace/sense electrodes and located in or adjacent the RV or the LV typically has a width of no more than 60 ms. as measured by a high impedance sense amplifier.

FIG 1 depicts an implanted, multi-channel cardiac pacemaker according to a first embodiment of the present invention for restoring AV synchronous contractions of the atrial and ventricular chambers and simultaneous or sequential pacing of the right and left ventricles. The pacemaker IPG 14 is implanted subcutaneously in a patient's body between the skin and the ribs. Three endocardial leads 16, 32 and 52 connect the IPG 14 with the RA, the RV and the LV, respectively. Each lead has at least one electrical conductor and pace/sense electrode, and a remote indifferent can electrode 20 is formed as part of the outer surface of the housing of the IPG 14. As described further below, the pace/sense electrodes and the remote indifferent can electrode 20 (IND_CAN electrode) can be selectively employed to provide a number of unipolar and bipolar pace/sense electrode combinations for pacing and sensing functions. The depicted positions in or about the right and left heart chambers are also merely exemplary. Moreover other leads and pace/sense electrodes may be used instead of the depicted leads and pace/sense electrodes that are adapted to be placed at electrode sites on or in or relative to the RA, LA, RV and LV.

The depicted bipolar endocardial RA lead 16 is passed through a vein into the RA chamber of the heart 10, and the distal end of the RA lead 16 is attached to the RA wall by an attachment mechanism 17. In the context of the illustrated embodiment of the present invention, the distal end of the lead 16 is located near the AV node or in the right atrial appendage, although other locations may be substituted in some cases. The bipolar endocardial RA lead 16 is formed with an in-line connector 13 fitting into a bipolar bore of IPG connector block 12 that is coupled to a pair of electrically insulated conductors within lead body 15 and connected with distal tip RA pace/sense electrode 19 and proximal ring RA pace/sense electrode 21. Delivery of atrial pace pulses and sensing of atrial sense events is effected between the distal tip RA pace/sense electrode 19 and proximal ring RA pace/sense electrode 21, wherein the proximal ring RA pace/sense electrode 21 functions as an indifferent electrode (IND_RA). Alternatively, a unipolar endocardial RA lead could be substituted for the depicted bipolar endocardial RA lead 16 and be employed with the IND_CAN electrode 20. Or, one of the distal tip RA pace/sense electrode 19 and proximal ring RA pace/sense electrode 21 can be employed with the IND_CAN electrode 20 for unipolar pacing and/or sensing.

Bipolar, endocardial RV lead 32 is passed through the vein and the RA chamber of the heart 10 and into the RV where its distal ring and tip RV pace/sense electrodes 38 and 40 are fixed in place in the apex by a conventional distal attachment mechanism 41. The RV lead 32 is formed with an in-line connector 34 fitting into a bipolar bore of IPG connector block 12 that is coupled to a pair of electrically insulated conductors within lead body 36 and connected with distal tip RV pace/sense electrode 40 and proximal ring RV pace/sense electrode 38, wherein the proximal ring RV pace/sense electrode 38 functions as an indifferent electrode (IND_RV).. Alternatively, a unipolar endocardial RV lead could be substituted for the depicted bipolar endocardial RV lead 32 and be employed with the IND_CAN electrode 20. Or, one of the distal tip RV pace/sense electrode 40 and proximal ring RV pace/sense electrode 38 can be employed with the IND_CAN electrode 20 for unipolar pacing and/or sensing.

In this illustrated embodiment, a unipolar, endocardial LV CS lead 52 is passed through a vein and the RA chamber of the heart 10, into the CS and then inferiorly in a branching vessel of the great vein 48 to extend the distal LV CS pace/sense electrode 50 alongside the LV chamber. The distal end of such LV CS leads is advanced through the superior vena cava, the right atrium, the ostium of the coronary sinus, the coronary sinus, and into a coronary vein descending from the coronary sinus, such as the great vein. The LV CS leads and LA CS leads may employ a deployable fixation mechanism or may instead rely on the close confinement within these vessels to maintain the pace/sense electrode or electrodes at a desired site. The LV CS lead 52 is formed with a small diameter single conductor lead body 56 coupled at the proximal end connector 54 fitting into a bore of IPG connector block 12. A small diameter unipolar lead body 56 is selected in order to lodge the distal LV CS pace/sense electrode 50 deeply in a vein branching inferiority from the great vein 48.

The distal, LV CS active pace/sense electrode 50 may be paired with the proximal ring RV indifferent pace/sense electrode 38 for delivering LV pace pulses across the bulk of the left ventricle and the intra-ventricular septum. The distal LV CS active pace/sense electrode 50 may also be paired with the distal tip RV active pace/sense electrode 40 for sensing across the RV and LV as described further below.

In an embodiment comprising a four chamber pacemaker, LV CS lead 52 could optionally bear a proximal LA CS pace/sense electrode positioned along the lead body and configured to lay in the larger diameter coronary sinus CS adjacent the LA. In that case, the lead body 56 would encase two electrically insulated lead conductors extending proximally from the more proximal LA CS pace/sense electrode(s) and terminating in a bipolar connector 54. The LV CS lead body may be smaller between the proximal LA CS electrode and the distal LV CS active pace/sense electrode 50. In that case, pacing of the RA would be accomplished along the pacing vector between the active proximal LA CS active electrode and the proximal ring RA indifferent pace/sense electrode 21.

Typically, in pacing systems of the type illustrated in FIG. 1, the electrodes designated above as "pace/sense" electrodes are used for both pacing and sensing functions. In accordance with one aspect of the present invention, these "pace/sense" electrodes can be selected to be used exclusively as pace or sense electrodes or to be used in common as pace/sense electrodes in programmed combinations for sensing cardiac signals and delivering pace pulses along pacing and sensing vectors. Separate or shared indifferent pace and sense electrodes can also be designated in pacing and sensing functions. For convenience, the following description separately designates pace and sense electrode pairs where a distinction is appropriate.

In addition, as described further below, each of the leads could carry a pressure sensor for developing systolic and diastolic pressures and a series of spaced apart impedance sensing leads for developing volumetric measurements of the expansion and contraction of the RA, LA, RV and LV.

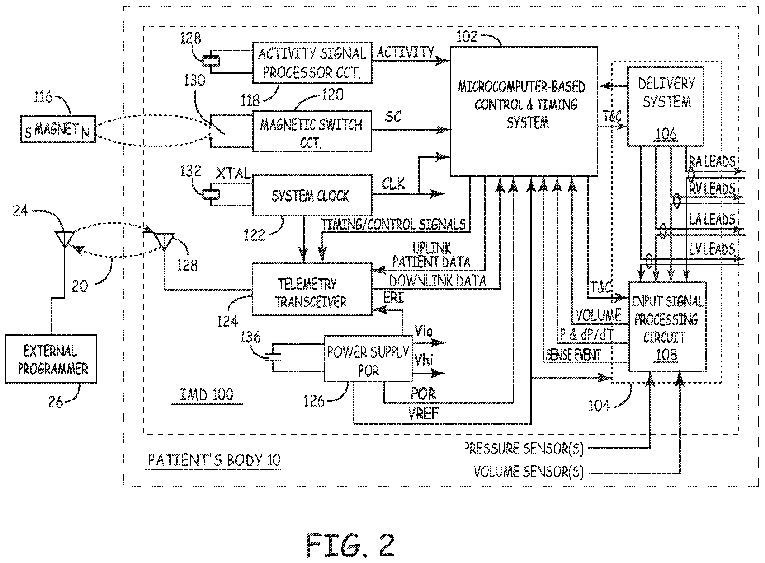

FIG. 2 depicts a system architecture of an exemplary multi-chamber pacemaker 100 implanted into a patient's body 10 that provides delivery of a therapy and physiologic input signal processing. A typical multi-chamber pacemaker 100 has a system architecture that is constructed about a microcomputer-based control and timing system 102 which varies in sophistication and complexity depending upon the type and functional features incorporated therein. The functions of microcomputer-based control and timing system 102 are controlled by firmware and programmed software algorithms stored in RAM and ROM including PROM and EEPROM and are carried out using a CPU, ALU, etc., of a typical microprocessor core architecture. The microcomputer-based multi-chamber stimulator/pacemaker control and timing system 102 may also include a watchdog circuit, a DMA controller, a block mover/reader, a CRC calculator, and other specific logic circuitry coupled together by on-chip data bus, address bus, power, clock, and control signal lines in paths or trees in a manner well known in the art. It will also be understood that control and timing of multi-chamber stimulator/pacemaker 100 can be accomplished with dedicated circuit hardware or state machine logic rather than a programmed micro-computer.

The multi-chamber pacemaker 100 also typically includes patient interface circuitry 104 for receiving signals from sensors and pace/sense electrodes located at specific sites of the patient's heart chambers and/or delivering stimulation to derive heart failure parameters or a pacing therapy to the heart chambers. The patient interface circuitry 104 therefore comprises a stimulation delivery system 106 optionally including pacing and other stimulation therapies and a physiologic input signal processing circuit 108 for processing the blood pressure and volumetric signals output by sensors. For purposes of illustration of the possible uses of the invention, a set of lead connections are depicted for making electrical connections between the therapy delivery system 106 and the input signal processing circuit 108 and sets of pace/sense electrodes located in operative relation to the RA, LA, RV and LV.

The therapy delivery system 106 can also optionally be configured to include circuitry for delivering cardioversion/defibrillation shocks and/or cardiac pacing pulses delivered to the heart or cardiomyostimulation to a skeletal muscle wrapped about the heart. The therapy delivery system 106 can also optionally be configured as a drug pump for delivering drugs into the heart to alleviate heart failure or to operate an implantable heart assist device or pump implanted in patients awaiting a heart transplant operation.

A battery provides a source of electrical energy to power the multi-chamber pacemaker operating system including the circuitry of multi-chamber pacemaker 100 and to power any electromechanical devices, e.g., valves, pumps, etc. of a substance delivery multi-chamber pacemaker, or to provide electrical stimulation energy of an ICD shock generator, cardiac pacing pulse generator, or other electrical stimulation generator. The typical energy source is a high energy density, low voltage battery 136 coupled with a power supply/POR circuit 126 having power-on-reset (POR) capability. The power supply/POR circuit 126 provides one or more low voltage power Vlo, the POR signal, one or more VREF sources, current sources, an elective replacement indicator (ERI) signal, and, in the case of an ICD, high voltage power Vhi to the therapy delivery system 106. Not all of the conventional interconnections of these voltages and signals are shown in FIG. 2.

In addition, in certain multi-chamber pacemakers, an audible patient alert warning or message is generated by a transducer 128 when driven by a patient alert driver 118 to advise of device operations, battery power level or a monitored patient condition. The patient may be warned of the detection of a malignant tachyarrhythmia and the imminent delivery of a cardioversion/defibrillation shock to enable the patient to assume a resting position prior to delivery.

Virtually all current electronic multi-chamber pacemaker circuitry employs clocked CMOS digital logic ICs that require a clock signal CLK provided by a piezoelectric crystal 132 and system clock 122 coupled thereto as well as discrete components, e.g., inductors, capacitors, transformers, high voltage protection diodes, and the like that are mounted with the ICs to one or more substrate or printed circuit board. In FIG. 2, each CLK signal generated by system clock 122 is routed to all applicable clocked logic via a clock tree. The system clock 122 provides one or more fixed frequency CLK signal that is independent of the battery voltage over an operating battery voltage range for system timing and control functions and in formatting uplink telemetry signal transmissions in the telemetry I/O circuit 124.

The RAM registers may be used for storing data compiled from sensed cardiac activity and/or relating to device operating history or sensed physiologic parameters for uplink telemetry transmission on receipt of a retrieval or interrogation instruction via a downlink telemetry transmission. The criteria for triggering data storage can also be programmed in via downlink telemetry transmitted instructions and parameter values The data storage is either triggered on a periodic basis or by detection logic within the physiologic input signal processing circuit 108 upon satisfaction of certain programmed-in event detection criteria. In some cases, the multi-chamber pacemaker 100 includes a magnetic field sensitive switch 130 that closes in response to a magnetic field, and the closure causes a magnetic switch circuit to issue a switch closed (SC) signal to control and timing system 102 which responds in a magnet mode. For example, the patient may be provided with a magnet 116 that can be applied over the subcutaneously implanted multi-chamber pacemaker 100 to close switch 130 and prompt the control and timing system to deliver a therapy and/or store physiologic episode data when the patient experiences certain symptoms. In either case, event related data, e.g., the date and time, may be stored along with the stored periodically collected or patient initiated physiologic data for uplink telemetry in a later interrogation session.

In the multi-chamber pacemaker 100, uplink and downlink telemetry capabilities are provided to enable communication with either a remotely located external medical device or a more proximal medical device on the patient's body or another multi-chamber pacemaker in the patient's body as described above with respect to Figs. 1 and 2. The stored physiologic data of the types described above as well as real-time generated physiologic data and non-physiologic data can be transmitted by uplink RF telemetry from the multi-chamber pacemaker 100 to the external programmer or other remote medical device 26 in response to a downlink telemetered interrogation command. The real-time physiologic data typically includes real time sampled signal levels, e.g., intracardiac electrocardiogram amplitude values, and sensor output signals. The non-physiologic patient data includes currently programmed device operating modes and parameter values, battery condition, device ID, patient ID, implantation dates, device programming history, real time event markers, and the like. In the context of implantable pacemakers and ids, such patient data includes programmed sense amplifier sensitivity, pacing or cardioversion pulse amplitude, energy, and pulse width, pacing or cardioversion lead impedance, and accumulated statistics related to device performance, e.g., data related to detected arrhythmia episodes and applied therapies. The multi-chamber pacemaker thus develops a variety of such real-time or stored, physiologic or non-physiologic, data, and such developed data is collectively referred to herein as "patient data".

The physiologic input signal processing circuit 108 therefore includes at least one electrical signal amplifier circuit for amplifying, processing and in some cases detecting sense events from characteristics of the electrical sense signal or sensor output signal. The physiologic input signal processing circuit 108 in multi-chamber pacemakers providing dual chamber or multi-site or multi-chamber monitoring and/or pacing functions includes a plurality of cardiac signal sense channels for sensing and processing cardiac signals from sense electrodes located in relation to a heart chamber. Each such channel typically includes a sense amplifier circuit for detecting specific cardiac events and an EGM amplifier circuit for providing an EGM signal to the control and timing system 102 for sampling, digitizing and storing or transmitting in an uplink transmission. Atrial and ventricular sense amplifiers include signal processing stages for detecting the occurrence of a P-wave or R-wave, respectively and providing an ASENSE or VSENSE event signal to the control and timing system 102. Timing and control system 102 responds in accordance with its particular operating system to deliver or modify a pacing therapy, if appropriate, or to accumulate data for uplink telemetry transmission or to provide a Marker Channel® signal in a variety of ways known in the art.

In addition, the input signal processing circuit 108 includes at least one physiologic sensor signal processing channel for sensing and processing a sensor derived signal from a physiologic sensor located in relation to a heart chamber or elsewhere in the body.

In the context of the present invention, the described pacemaker provides the inventive stimulation therapy to the atrial electrodes at defined time intervals during the defined time window discussed above, following a normally conducted ventricular depolarization. For purposes of the present application, a normally conducted ventricular depolarization should be understood to be a ventricular depolarization preceded by an atrial depolarization at an interval corresponding to a normal A-V conduction interval (i.e. not a premature ventricular depolarization (PVC)). The ventricular depolarization may be sensed, paced or some combination of both, (e.g. during delivery of ventricular resynchronization therapy).

Activation of the stimulation therapy of the present invention may be triggered by the device, for example in response to an excessive heart rate or blood pressure and the stimulation therapy may be deactivated responsive to the heart rate or blood pressure returning to acceptable ranges. The determination of whether the heart rate or blood pressure is excessive may be made by comparing the values of these parameters to pre-defined threshold values. Alternatively, activation and deactivation of the stimulation therapy of the present invention may be triggered by the patient or attending physician by means of an associated programmer or activator.

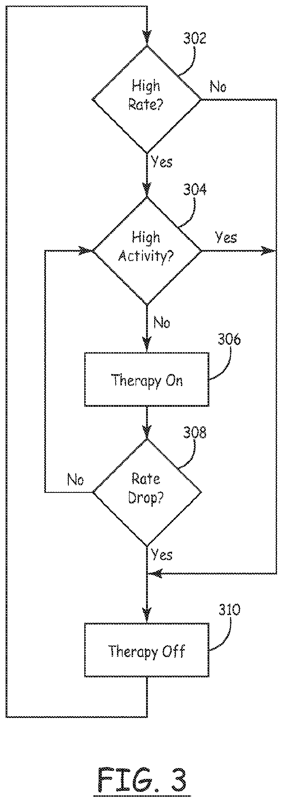

Figure 3 is a functional flowchart illustrating the operation of a pacemaker practicing a first embodiment of the present invention. The operation of the pacemaker to provide the therapy is controlled by the microprocessor responsive to software stored in memory, comprising instructions for causing the pacemaker to perform the described functions. The pacemaker checks at 302 to determine whether the heart rate is elevated, e.g. by determining the rate is above a defined threshold. Alternatively or additionally, the pacemaker may cross check the present rate at 304 with a physiological sensor such as an activity sensor to determine whether the heart rate is appropriate. If the heart rate is determined to be excessive stimulation therapy according to the present invention is initiated at 306. The therapy is continued until either the ventricular rate drops below a defined threshold at 308 (optionally also defined relative to the output of the physiological sensor) or until the output of the physiologic sensor indicates a higher heart rate is appropriate at 304. Following either of these events, stimulation therapy according to the present invention may be terminated at 310.

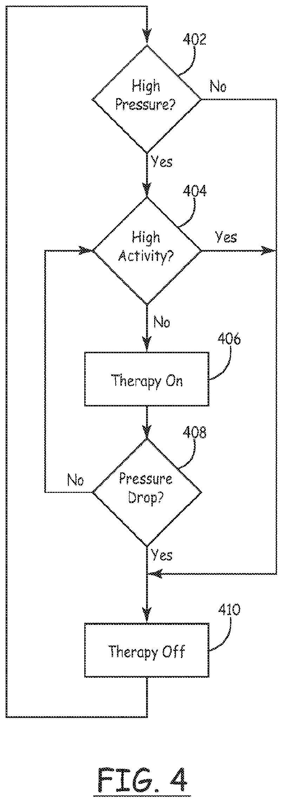

Figure 4 is a functional flowchart illustrating the operation of a pacemaker practicing a second embodiment of the present invention, in this case responsive to a sensed inappropriately high blood pressure. The operation of the pacemaker to provide the therapy is similarly controlled by the microprocessor responsive to software stored in memory, comprising instructions for causing the pacemaker to perform the described functions. The pacemaker checks at 402 to determine whether the blood pressure is elevated, e.g. by determining the pressure is above a defined threshold. Alternatively or additionally, the pacemaker may cross check the present pressure at 404 with a physiological sensor such as an activity sensor to determine whether the blood pressure is appropriate. If the blood pressure is determined to be excessive stimulation therapy according to the present invention is initiated at 406. The therapy is continued until either the blood pressure drops below a defined threshold at 408 (optionally also defined relative to the output of the physiological sensor) or until the output of the physiologic sensor indicates a higher blood pressure is appropriate at 404. Following either of these events, stimulation therapy according to the present invention may be terminated at 410.

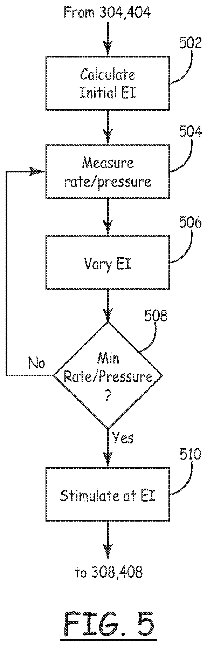

Figure 5 is a functional flow chart illustrating details of operation of a pacemaker practicing the first or second embodiments of the present invention discussed above. In particular, Figure 5 illustrates a mechanism for automatically adjusting the timing and/or amplitude of the stimulation provided by the present invention. At 502, stimulation is initiated and an initial escape interval is defined. The escape interval (EI) may be timed from a preceding atrial and/or ventricular depolarization as discussed above. The initial value of EI may be chosen, for example, to fall near the middle of the defined stimulation time window as discussed above, or near the beginning or ending of the window. At 504, the pacemaker measures the rate and/or blood pressure. The pacemaker then varies the escape interval (EI) by increasing it or decreasing it at 506 and continuing to do so until a desired result is achieved at 508 and thereafter stimulates at those parameters at 510. The desired result, as discussed above, may be a minimum obtainable level for heart rate or blood pressure or a heart rate or blood pressure below a defined threshold, or as close as possible to either one. As noted above, the defined threshold pressure or rate may be determined based upon the output of a physiological sensor such as an activity sensor. Variation of the duration of EI can be accomplished by means of gradual increase or decrease of El or by means of a binary search type operation.

In most embodiments, it is anticipated that the parameters of the individual stimulation pulses (width, amplitude, etc.) will be determined at implant and programmed into the pacemaker's memory by the attending physician. However, in some embodiments mechanism for optimizing these parameters analogous to the mechanism described above in conjunction with Figure 5 may be employed.

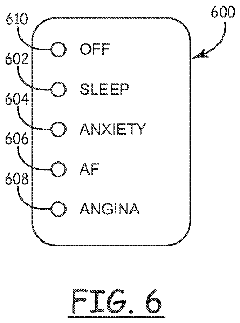

Figure 6 illustrates an external activator or programmer 600 which may be used by the patient to activate the stimulation therapy of the present invention responsive to the occurrence of symptoms. In simpler embodiments, the patient may simply be provided with on and off buttons to activate and deactivate therapy as needed. In more complex embodiments, particular stimulation parameters may be pre-programmed for activation depending upon the symptoms being experienced. For example, the activator may be provided with a menu of symptoms and associated control buttons or other inputs associate with difficulty in sleeping (602), Anxiety (604) atrial fibrillation or flutter (606) or angina (608). The patient preferably will be able to deactivate the therapy at any time using an off button or control 610 at any time, including delivery of the therapy as automatically triggered by the implantable pacemaker itself.

## Claims

1. An apparatus for reducing a patient's heart rate or blood pressure, comprising:
sense amplifiers responsive to atrial and ventricular depolarizations having associated refractory periods thereafter;
a generator (106) providing stimulus pulses; and
circuitry (102) for activation of a stimulation therapy, the stimulation therapy comprising triggering the pulse generator responsive to detection of an occurrence of a ventricular depolarization following a preceding atrial depolarization; **characterized by** the apparatus including means for defining a time window following the ventricular depolarization, the means for defining the time window determining expiry of the refractory period of the stimulated atrial and/or nodal tissue and expiry of the refractory period of the ventricle and defining the time window as the period between expiry of the respective refractory periods; and means to control timing of delivery of a stimulus pulse to the patient's atrial and/or nodal tissue to be within the defined time window and thus within the associated refractory period of the ventricle but outside of an associated refractory period of the stimulated atrial and/or nodal tissue; the means to deliver a stimulus pulse being configured to initiate stimulation and to define an escape interval relative to the defined time window.

2. The apparatus of claim 1, further comprising:
a processor (108) configured to determine the patient's heart rate; wherein said circuitry (102) is responsive to the patient's determined heart rate exceeding a predetermined rate for activation of the stimulation therapy.

3. The apparatus of claim 1, further comprising:
a blood pressure sensor; and
a processor (108) responsive to the patient's blood pressure; and wherein
said circuitry (102) is responsive to the patient's determined blood pressure exceeding a predetermined value for activation of said stimulation therapy.

## Patentansprüche

1. Gerät zum Reduzieren einer Herzfrequenz oder eines Blutdruckes eines Patienten, umfassend:
Wahrnehmungsverstärker, die ansprechend auf atriale und ventrikuläre Depolarisationen mit anschließenden assoziierten refraktären Perioden sind;
einen Generator (106), der Stimuluspulse bereitstellt; und
eine Schaltung (102) zur Aktivierung einer Stimulationstherapie, wobei die Stimulationstherapie ein Triggern des Pulsgenerators ansprechend auf eine Erfassung eines Auftretens einer ventrikulären Depolarisation, die einer vorhergehenden atrialen Depolarisation folgt, umfasst; **dadurch gekennzeichnet, dass** das Gerät ein Mittel zum Definieren eines Zeitfensters, das der ventrikulären Depolarisation folgt, umfasst, wobei das Mittel zum Definieren des Zeitfensters einen Ablauf der refraktären Periode des stimulierten atrialen und/oder nodalen Gewebes und einen Ablauf der refraktären Periode des Ventrikels umfasst, und das Zeitfenster als die Periode zwischen Ablauf der jeweiligen refraktären Perioden definiert; und ein Mittel zum Steuern eines Timings einer Abgabe eines Stimuluspulses an das atriale und/oder nodale Gewebe innerhalb des definierten Zeitfensters und daher innerhalb der assoziierten refraktären Periode des Ventrikels, aber außerhalb einer assoziierten refraktären Periode des stimulierten atrialen und/oder nodalen Gewebes ist; wobei das Mittel zum Abgeben eines Stimuluspulses konfiguriert ist, um eine Stimulation einzuleiten, und ein Escape- bzw. Ausstiegsintervall relativ zu dem definierten Zeitfenster zu definieren.

2. Gerät nach Anspruch 1, ferner umfassend:
einen Prozessor (108), der konfiguriert ist, um die Herzfrequenz des Patienten zu bestimmen; wobei die Schaltung (102) ansprechend darauf ist, dass die bestimmte Herzfrequenz des Patienten eine vorbestimmte Rate zur Aktivierung der Stimulationstherapie überschreitet.

3. Gerät nach Anspruch 1, ferner umfassend:
einen Blutdrucksensor; und
einen Prozessor (108), der ansprechend auf den Blutdruck des Patienten ist; und
wobei die Schaltung (102) ansprechend darauf ist, dass der Blutdruck des Patienten einen vorbestimmten Wert zur Aktvierung der Stimulationstherapie überschreitet.

## Revendications

1. Appareil destiné à réduire la fréquence cardiaque ou la pression sanguine d'un patient, comportant :
des amplificateurs de détection sensibles à des dépolarisations auriculaires et ventriculaires ayant des périodes réfractaires associées après celles-ci ;
un générateur (106) délivrant des impulsions de stimulus ; et
des circuits (102) pour l'activation d'une thérapie de stimulation, la thérapie de stimulation comportant le déclenchement du générateur d'impulsions en réponse à la détection d'une occurrence d'une dépolarisation ventriculaire après une dépolarisation auriculaire précédente ; **caractérisé en ce que** l'appareil inclut des moyens pour définir une fenêtre de temps après la dépolarisation ventriculaire, les moyens pour définir la fenêtre de temps déterminant l'expiration de la période réfractaire du tissu auriculaire et/ou nodal stimulé et l'expiration de la période réfractaire du ventricule et définissant la fenêtre de temps comme la période comprise entre l'expiration des périodes réfractaires respectives ; et des moyens pour commander la cadence de délivrance d'une impulsion de stimulus au tissu auriculaire et/ou nodal du patient pour qu'elle soit dans la fenêtre de temps définie et ainsi dans la période réfractaire associée du ventricule mais en dehors d'une période réfractaire associée du tissu auriculaire et/ou nodal stimulé ; les moyens pour délivrer une impulsion de stimulus étant configurés pour déclencher une stimulation et pour définir un intervalle d'échappement par rapport à la fenêtre de temps définie.

2. Appareil selon la revendication 1, comportant en outre : un processeur (108) configuré pour déterminer la fréquence cardiaque du patient ; dans lequel lesdits circuits (102) sont sensibles à la fréquence cardiaque déterminée du patient dépassant une fréquence prédéterminée pour l'activation d'une thérapie de stimulation.

3. Appareil selon la revendication 1, comportant en outre :
un capteur de pression sanguine ; et
un processeur (108) sensible à la pression sanguine du patient ; et dans lequel
lesdits circuits (102) sont sensibles à la pression sanguine déterminée du patient dépassant une valeur prédéterminée pour l'activation de ladite thérapie de stimulation.
